# EUROPÄISCHE PATENTSCHRIFT

(11) **EP 1 141 583 B1**
(45) Veröffentlichungstag und Bekanntmachung des Hinweises auf die Patenterteilung: **30.07.2003**
(21) Anmeldenummer: 99959636.4
(22) Anmeldetag: 29.12.1999
(51) Int. Cl.: F16H 31/00

(54) **ANTRIEB FÜR EINEN ROTIERENDEN GEGENSTAND, WIE EINE WALZE, WELLE, PLATTE ODER DERGLEICHEN**
DRIVE MECHANISM FOR A ROTATING OBJECT SUCH AS A ROLLER, SHAFT, DISK OR SUCH LIKE
ENTRAINEMENT POUR UN OBJET TOURNANT, TEL QU'UN CYLINDRE, UN ARBRE, UN DISQUE OU ANALOGUE

(30) Priorität: 29.12.1998 US 222726
(43) Veröffentlichungstag der Anmeldung: 10.10.2001
(73) Patentinhaber: Venta Airwasher LLC., Elk Grove Village, IL 60007 (US); Venta-Luftwäscher GmbH, 88250 Weingarten (DE)
(72) Erfinder: HARTER, Erich, D-88284 Mochenwangen (DE); HITZLER, Alfred, D-88284 Mochenwangen (DE)
(74) Vertreter: Otten, Herbert
(86) Internationale Anmeldenummer: IB9902063
(87) Internationale Veröffentlichungsnummer: WO00039485

(56) Entgegenhaltungen:
- EP-A- 0 838 610
- FR-A- 989 820
- FR-A- 2 595 839

## Beschreibung

### Stand der Technik

Die Erfindung betrifft einen Antrieb für einen rotierenden Gegenstand nach dem Oberbegriff des Anspruchs 1.

Ein solcher Antrieb ist bereits durch die europäische Offenlegungsschrift EP 083 8610 A2 bekanntgeworden. Das Kernstück dieses Antriebs ist mindestens ein schwenkbar gelagerter Hebel, der mit einem Zahnrad zusammenwirkt, das drehfest mit dem rotierenden Gegenstand verbundenen ist. Hierzu ist eine Vorrichtung zur Lagerung und Führung des Hebels für eine in das Zahnrad eingreifende und mitnehmende Vorwärtsbewegung des Hebels vorhanden.

Dieser Antrieb hat den Vorteil, daß er im Wesentlichen unempfindlich gegenüber Verunreinigungen ist, die durch eine Flüssigkeit auftreten, in welcher Walzen, Wellen oder dergleichen in einem Luftbefeuchtungs- und Reinigungsgerät, einem Duftverdunster oder dergleichen rotiert. Denn selbst wenn das Zahnrad hierbei durch die Rotationsbewegung in der Flüssigkeit von dieser benetzt wird, besteht der einzige Berührungspunkt mit den weiteren Getriebekomponenten in dem Teil des Hebels, der in das Zahnrad eingreift. Dieser Teil des Hebels ist jedoch in der Regel einfach zu reinigen.

Ein Nachteil dieses Mechanismus besteht jedoch darin, daß insbesondere durch Auftreten von Fertigungstoleranzen der Antriebsteile ein gleichmäßiger geräuscharmer Lauf des rotierenden Gegenstandes mitunter beeinträchtigt ist, da insbesondere der im Eingriff stehende Hebel mit dem Zahnrad verhaken kann.

### Aufgabe und Vorteile der Erfindung

Der Erfindung liegt die Aufgabe zugrunde einen Antrieb zu schaffen, der einen verbesserten Gleichlauf aufweist und insbesondere unempfindlicher gegenüber Fertigungstoleranzen ist.

Diese Aufgabe wird durch die Merkmale des Anspruchs 1 gelöst.

In den Unteransprüchen sind vorteilhafte und zweckmäßige Weiterbildungen des erfindungsgemäßen Antriebs angegeben.

Die Erfindung geht von einem Antrieb für einen rotierenden Gegenstand für eine Walze, Welle, Platte oder dergleichen, insbesondere für einen Duftverdunster, einen Luftbefeuchter oder ein Luftreinigungsgerät oder dergleichen aus, das die folgenden Merkmale aufweist:

Einen beweglich gelagerten Hebel, ein Zahnrad, das drehfest mit dem rotierenden Gegenstand verbunden ist sowie ein Hebelgetriebe für eine in das Zahnrad eingreifende und mitnehmende Bewegung des Hebels zur Drehung des Zahnrades, wobei der Hebel zum Eingriff in die Zähne des Zahnrads eine Spitze aufweist. Der Kerngedanke der Erfindung liegt nun darin, daß die Spitze in Form eines Zahnes ausgebildet ist, wobei die Form dieses Zahnes und die der Zähne des Zahnrades sowie das Hebelgetriebe derart aufeinander abgestimmt sind, daß die Zahnflanken des Zahnes am wenigstens einen Hebel und des jeweils im Eingriff stehenden Zahnes am Zahnrad bei der mitnehmenden Bewegung aufeinander abwälzen. Durch diese Maßnahme wird erreicht, daß der Antrieb auch bei Auftreten von Fertigungstoleranzen das Zahnrad und den rotierenden Gegenstand in eine gleichmäßige Bewegung versetzt, wobei ein Verhaken von Hebelspitze und Zahnrad vermieden werden kann. Denn durch die erfindungsgemäße Ausgestaltung werden im Wesentlichen die Gesetzmäßigkeiten einer herkömmlichen Verzahnung zwischen zwei Zahnrädern erfüllt. Hierdurch wird außerdem der Verschleiß der sich berührenden Zahnflanken im Vergleich zur Lösung aus dem Stand der Technik deutlich verbessert, da die sonst vorhandene Gleitbewegung zwischen den Flanken stark herabgesetzt ist, und im Wesentlichen durch eine Abrollbewegung ersetzt wird. Dies macht sich insbesondere bei der Lebensdauer des Antriebs und beim Betriebsgeräusch positiv bemerkbar.

Um den Gleichlauf des Antriebs noch weiter zu verbessern, wird überdies vorgeschlagen, daß die Form des Zahnes am Hebel und die der Zähne des Zahnrades sowie das Hebelgetriebe derart aufeinander abgestimmt sind, daß die Zahnflanken des Zahnes am wenigstens einen Hebel und des jeweils im Eingriff stehenden Zahnes am Zahnrad bei der mitnehmenden Bewegung einen Berührungspunkt aufweisen, in dem die Flanken ohne radiales Gleiten aufeinander abrollen (Wälzpunkt).

Vorzugsweise ist die Form des Zahnes am Hebel und die der Zähne am Zahnrad nach den Regeln der Verzahnung als Evolventen-, Zykloiden-, Kreisbogen- oder als Triebstockverzahnung, bei welcher der Zahn am Hebel z.B. die Gestalt eines Zapfens besitzt, ausgebildet. Auf diese Weise läßt sich ein im Wesentlichen konstantes Übersetzungsverhältnis zwischen Hebelgetriebe und Zahnrad realisieren.

In einer besonders vorteilhaften Ausgestaltung der Erfindung beinhaltet das Hebelgetriebe zur Schubführung des Hebels Führungsflächen für den Hebel. Dadurch kann der Hebel geführt beispielsweise eine Vorwärtsbewegung, eine Rückwärtsbewegung sowie eine Schwenkbewegung durchführen. In diesem Zusammenhang ist es vorteilhaft, wenn das Hebelgetriebe einen Exzenterantrieb für den Hebel umfaßt. Durch eine entsprechend aufeinander abgestimmte geometrische Anordnung der Gelenkpunkte des Hebels und der Führungsflächen am Hebelgetriebegehäuse kann für den zyklischen Eingriff der zahnförmigen Spitze des wenigstens einen Hebels eine gewünschte Koppelkurve erreicht werden. Anstatt des dadurch realisierten Funktionsprinzips des Schubkurbelgetriebes kann in einer anderen Ausführungsform auch das Funktionsprinzip der Kurbelschleife angewandt werden, bei welchem sich die Längsführung im Hebel befindet, in die beispielsweise ein im Hebelgetriebegehäuse festgelegter Zapfen eingreift.

Zur Realisierung eines einfachen Exzenterantriebs wird im weiteren vorgeschlagen, daß dieser eine Antriebswelle für den Hebel aufweist, die exzentrisch auf einem Zahnrad angeordnet ist. Das Zahnrad kann kontinuierlich angetrieben werden, vorzugsweise mit Hilfe einer Schnecke. Hierdurch sind zum einen extreme Übersetzungen möglich, was insbesondere bei der Verwendung in einem erfindungsgemäßen Duftverdunster oder Luftwäscher, wo nur ein Motor zum direkten Antrieb des Ventilators und Antrieb eines oder zweier Plattenstapel eingesetzt wird, notwendig ist. Zum anderen stellt ein Schneckengetriebe ein sogenanntes selbsthemmendes Getriebe dar, wodurch der wenigstens eine Hebel gegen eine unerwünschte Bewegung blockiert ist.

In einer bevorzugten Ausführungsform wird die Antriebswelle direkt exzentrisch auf einem Zahnrad angebracht, welches von einer Schnecke angetrieben wird.

Vorzugsweise wird die Schnecke über eine elastische Kupplung mit der Antriebswelle eines Motors verbunden. Hierdurch braucht die Antriebswelle nicht vollständig exakt in Flucht mit der Schneckenwelle zu stehen. Größere Toleranzen bei der Fertigung eines erfindungsgemäßen Antriebs sind hierbei möglich, was unter anderem eine Kostenersparnis mit sich bringt.

In einer besonders vorteilhaften Ausgestaltung der Erfindung sind zwei, drei oder mehr Hebel vorgesehen, die abwechselnd in das Zahnrad eingreifen. Dadurch befindet sich ständig mindestens ein Hebel im Eingriff mit dem Zahn- bzw. Zapfenkranz des rotierenden Gegenstandes, so daß dieser gleichmäßig angetrieben wird. Des Weiteren wird insbesondere für den Fall gewährleistet, daß die Hebel durch ein Schneckengetriebe gegen eine unerwünschte Bewegung verriegelt sind, daß auch der rotierende Gegenstand durch wenigstens einen im Eingriff befindlichen Hebel gegen jede unerwünschte weitere Bewegung, beispielsweise gegen ein Zurückdrehen gesichert ist. Die Rotation des rotierenden Gegenstandes ist somit ständig mechanisch fest durch den Hebelantrieb kontrolliert.

Im weiteren ist es besonders bevorzugt, wenn der wenigstens eine Hebel zwischen den Führungsflächen des Hebelgetriebes elastisch gelagert ist. Dadurch wird der Hebel im Hebelgetriebe vorgespannt und weist auch bei Fertigungstoleranzen kein Spiel auf. Ein zu großes Spiel könnte dazu führen, daß im schlimmsten Fall der Zahn eines eingreifenden Hebels mit einem Zahn des Zahnrades kollidiert oder sich die Zähne verhaken.

Besonders bevorzugt ist es dabei, wenn zur elastischen Lagerung des Hebels zwischen den Führungsflächen wenigstens ein Teil der von den Führungsflächen erfaßten Außenkontur des Hebels elastisch nachgiebig ist. In diesem Zusammenhang ist es vorteilhaft, wenn zur Realisierung einer elastisch nachgiebigen Außenkontur des Hebels, die Außenkontur als schmaler Rahmen ausgebildet ist. Insbesondere, wenn der Hebel aus Kunststoff hergestellt ist, weist dadurch dieser Konturbereich federnde Eigenschaften auf.

Um die Federeigenschaften noch zu verbessern, ist es darüber hinaus bevorzugt, wenn der Rahmen unterbrochen ist, wobei in die Unterbrechungsstelle ein Federelement eingesetzt ist.

Um eine effektive Kraftübertragung des wenigstens einen Hebels auf das Zahnrad zu gewährleisten, wird schließlich vorgeschlagen, daß der Bereich der Außenkante des Hebels nicht elastisch nachgiebig ist, der sich zur Aufnahme der wesentlichen Kraft für eine Weiterbewegung des Zahnrades an einer Führungsfläche des Hebelgetriebes abstützt.

### Zeichnungen

Mehrere Ausführungsbeispiele der Erfindung sind in den Zeichnungen dargestellt und in der nachfolgenden Beschreibung unter Angabe weiterer Vorteile und Einzelheiten näher erläutert. Es zeigen
- Fig. 1: eine Seitenansicht von zwei nebeneinander angeordneten Plattenstapel eines Luftbefeuchtungsgerätes mit jeweils daran angeordneten erfindungsgemäßen Antrieben in einer schematischen Seitenansicht,
- Fig. 2a-e: einen Ausschnitt eines erfindungsgemäßen Antriebs gemäß Fig. 1 im schematischen Querschnitt für verschiedene Hebelpositionen,
- Fig. 3a-c: einen erfindungsgemäßen Hebel in zwei Draufsichten mit und ohne Federelement sowie einer Seitenansicht,
- Fig. 4: ein Schneckenrad mit exzentrisch angeordneter Antriebswelle für einen Hebel gemäß Fig. 3a bis c in einer schematischen Draufsicht,
- Fig. 5a: einen Ausschnitt eines erfindungsgemäßen Antriebs entsprechend der Figuren 2a bis e jedoch mit drei Antriebshebeln für eine Position und
- Fig. 5b: einen erfindungsgemäßen Antrieb gemäß Fig. 5a im Längsschnitt.

### Beschreibung der Ausführungsbeispiele

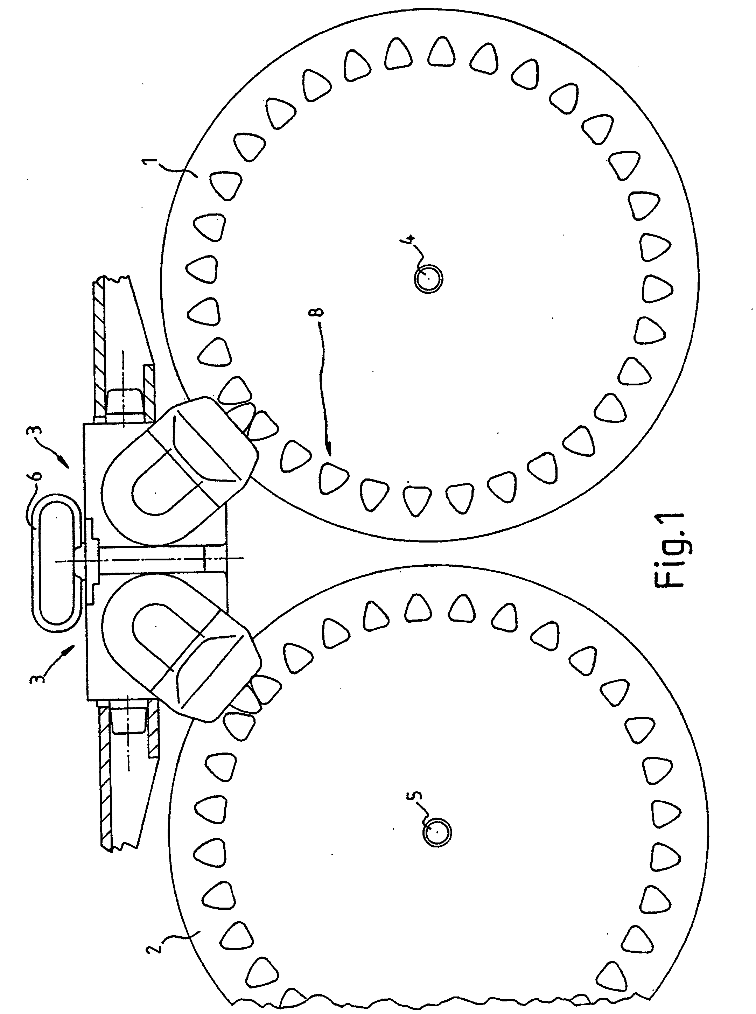

In Fig. 1 sind zwei parallel gelagerte Plattenstapel 1, 2 eines Luftbefeuchtungsgerätes mit daran angeordneten erfindungsgemäßen Antrieben 3 in einer schematischen Seitenansicht dargestellt. Die Plattenstapel 1, 2 bestehen z. B. aus lammellenförmig hintereinander angeordneten kreisförmigen Scheiben. Die Plattenstapel 1, 2 sind im Luftwäscher normalerweise an einer Wanne gelagert (nicht dargestellt), in der sich in der Regel Wasser, gegebenenfalls mit einem Zusatzstoff befindet, in welches die Plattenstapel bei einer Drehung um Achsen 4, 5 eintauchen. Ebenfalls nicht dargestellt ist ein Gehäusedeckel an dem z. B. ein Ventilator mit Antriebsmotor angeordnet ist, der über eine elastische Kupplung 6 mit beiden erfindungsgemäßen Antrieben 3 in Verbindung steht.

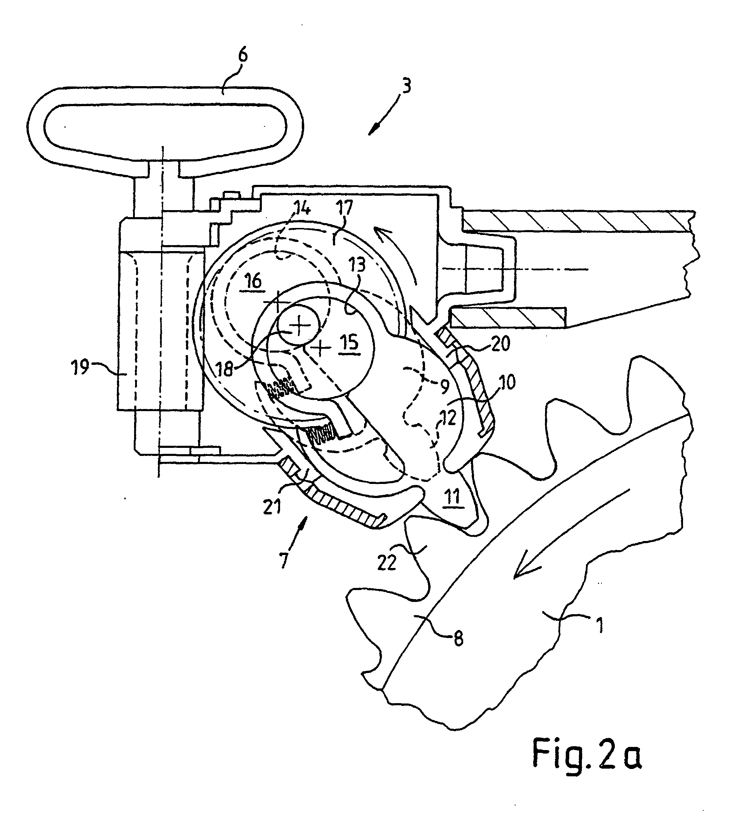

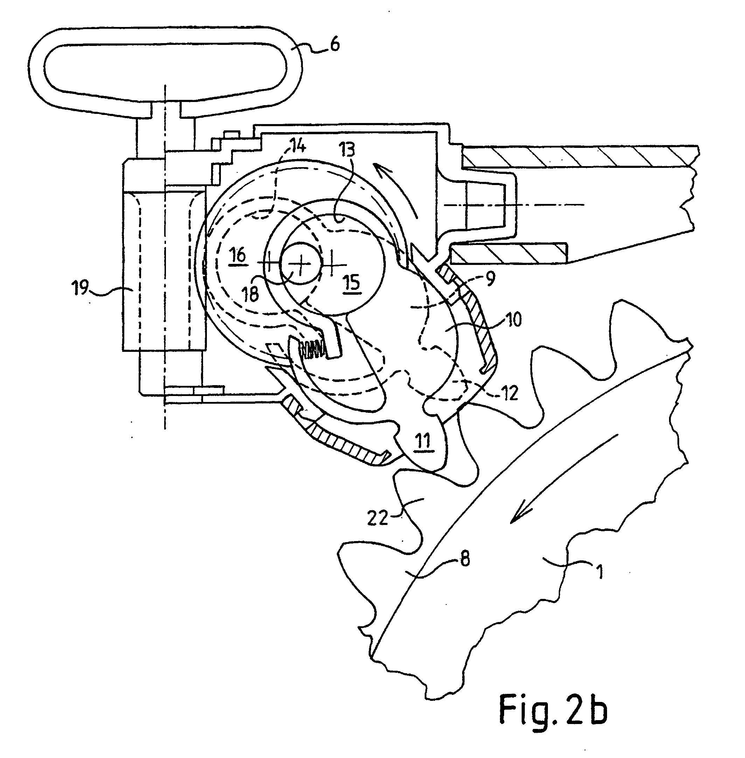

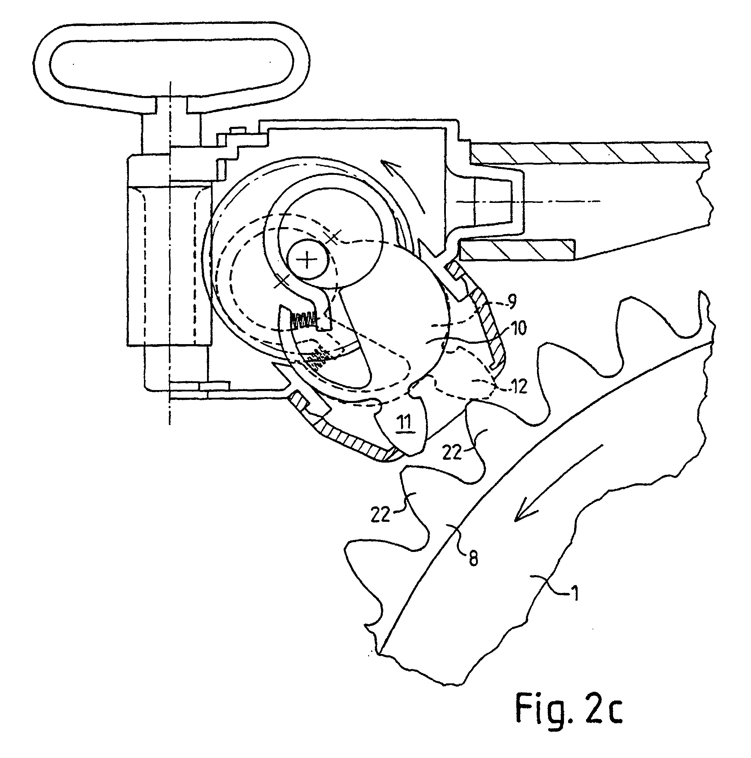

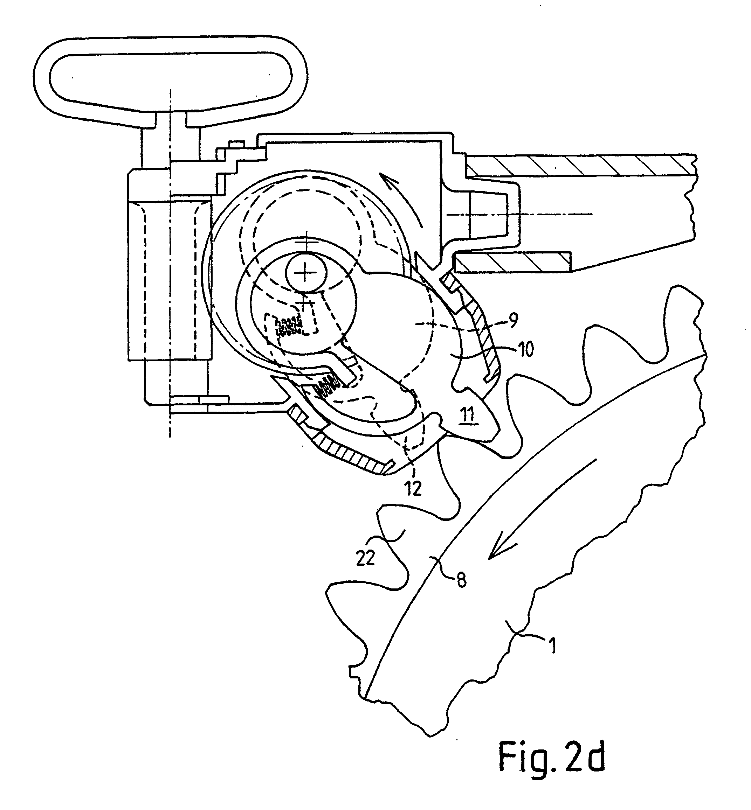

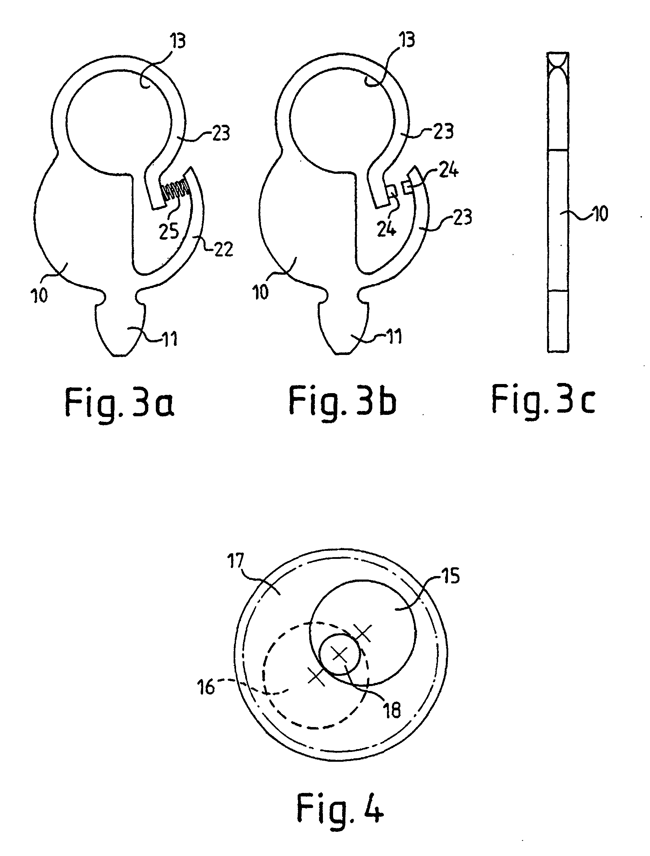

In den Fig. 2a der e ein der erfindungsgemäßer Antrieb 3 ausschnittsweise vergrößert und im Schnittbild für verschiedene Hebelpositionen dargestellt. Diese Figuren zeigen ein Hebelgetriebe 7, das mit einem Zahnkranz 8 zusammenwirkt, der an der Stirnseite des Plattenstapels 1 angeordnet ist. Bei dem Hebelgetriebe 7 handelt es sich um eine Anordnung mit zwei Antriebshebeln 9, 10. Die Antriebshebel 9, 10 besitzen jeweils eine zahnförmig ausgestaltete Spitze 11, 12 sowie eine Aufnahmeöffnung 13, 14 (siehe auch Fig. 3a und b) für jeweils zwei Antriebszapfen 15, 16. Die Antriebszapfen 15, 16 sind an den beiden Stirnflächen eines Schneckenrades 17 exzentrisch um 180° versetzt angebracht und bilden somit einen exzentrischen Lagersitz für die darauf über die Aufnahmeöffnungen 13, 14 aufgesteckten Antriebshebel 9, 10. Das Schneckenrad 17 besitzt eine Drehachse 18, die im Gehäuse des Hebelgetriebes 7 gelagert ist und steht mit einer Schnecke 19 in Eingriff. Die Schnecke 19 ist über die elastische Kupplung 6 mit z.B. einem nicht gezeigten Antriebsmotor verbunden. Bei einer Drehung der Schnecke 19 und einer damit bewirkten Drehung des Schneckenrades 17 z.B. in Pfeilrichtung werden über den exzentrischen Lagersitz 13, 14, 15, 16 der Antriebshebel 9, 10 diese im Gehäuse des Hebelgetriebes 7 vor und zurück bewegt bzw. geschwenkt. Dabei liegt die Außenkontur der Antriebshebel 9, 10 an Führungsflächen 20, 21 des Gehäuses des Hebelgetriebes 7 an.

In Fig. 2a ist der vordere Antriebshebel 10 (in durchgezogenen Linien dargestellt) im Eingriff mit der Verzahnung des Zahnkranzes 8, während der zweite dahinter angeordnete Antriebshebel 9 (in gestrichelten Linien dargestellt) völlig aus der Verzahnung des Zahnkranzes 8 zurückgenommen ist. Die gewünschte Bewegung der zahnförmigen Spitzen 11, 12 der Antriebshebel 9, 10 ergibt sich im Wesentlichen aus den geometrischen Abständen der exzentrisch angeordneten Antriebszapfen 15, 16 sowie der Form und dem Abstand der Führungsflächen 20, 21. Die Bahnkurve der zahnförmigen Spitzen 11, 12 stellt sich als Koppelkurve des somit realisierten Schubkurbelantriebs dar.

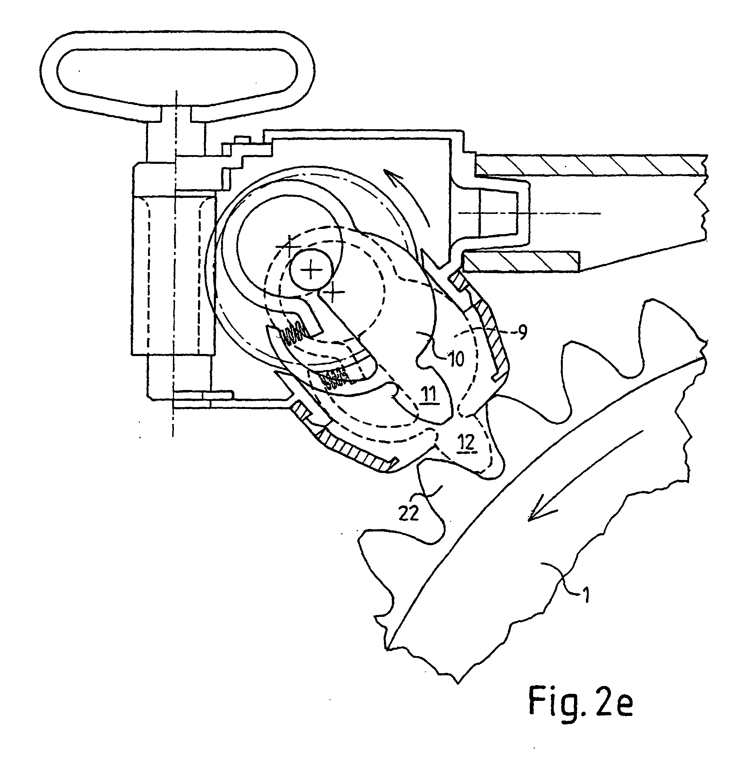

Die Bewegung der Antriebshebel 9, 10 zur Erzielung einer Drehbewegung des am Plattenstapel 1 angeordneten Zahnkranzes 8 in Pfeilrichtung ist in unterschiedlichen Winkelpositionen des Schneckenrades 17 in den Figuren 2a bis e abgebildet. Dabei entspricht jede Figur einem Zustand der Hebelposition für eine Drehung des Schneckenrades 17 um 45°. D.h. Fig. 2e stellt eine Drehung um 180° dar, sofern Fig. 2a mit 0° festgelegt wird.

Die Form von Zähnen 22 des Zahnkranzes 8 sowie der zahnförmigen Spitze 11, 12 ist so ausgebildet, daß die ineinander greifenden Zähne beispielhaft eine Zykloidenverzahnung realisieren. Außerdem ist die Bewegung der Antriebshebel 9, 10 durch die exzentrische Lagerung sowie die Führungsflächen am Gehäuse des Hebelgetriebes so abgestimmt, daß die Zahnflanken aufeinander abwälzen.

In den Figuren 3a bis c ist beispielhaft der Antriebshebel 10 nochmals im einzelnen dargestellt. Die Außenkontur des Antriebshebels 10 ist über einen großen Bereich auf einer Seite als schmaler Rahmen 23 ausgebildet. Vor der Aufnahmeöffnung 13 ist der Rahmen 23 unterbrochen und weist Aufnahmezapfen 24 für eine Druckfeder 25 (nur in Fig. 3a dargestellt) auf. Auf diese Weise ist der vordere Bereich des Antriebshebels 10 auf dieser Seite elastisch nachgiebig. Dies ermöglicht, den Antriebshebel 10 oder dem gleich gestalteten Antriebshebel 9 mit Vorspannung in das Gehäuse des Hebelgetriebes 7 einzusetzen. Damit wird verhindert, daß bei auftretenden Fertigungstoleranzen die Hebel 9, 10 ein Spiel aufweisen, daß gegebenenfalls dazu führt, daß die Spitze 11, 12 eines Antriebshebels 9, 10 mit einem Zahn des Zahnkranzes 8 kollidiert oder verhakt. Durch die unsymmetrische Rahmenbildung wird erreicht, daß sich der Hebel an einer Führungsfläche ohne nachzugeben abstützen kann. Dies ist vorzugsweise die Seite, die die wesentlichen Kräfte für die Weiterbewegung des Zahnrades aufnimmt.

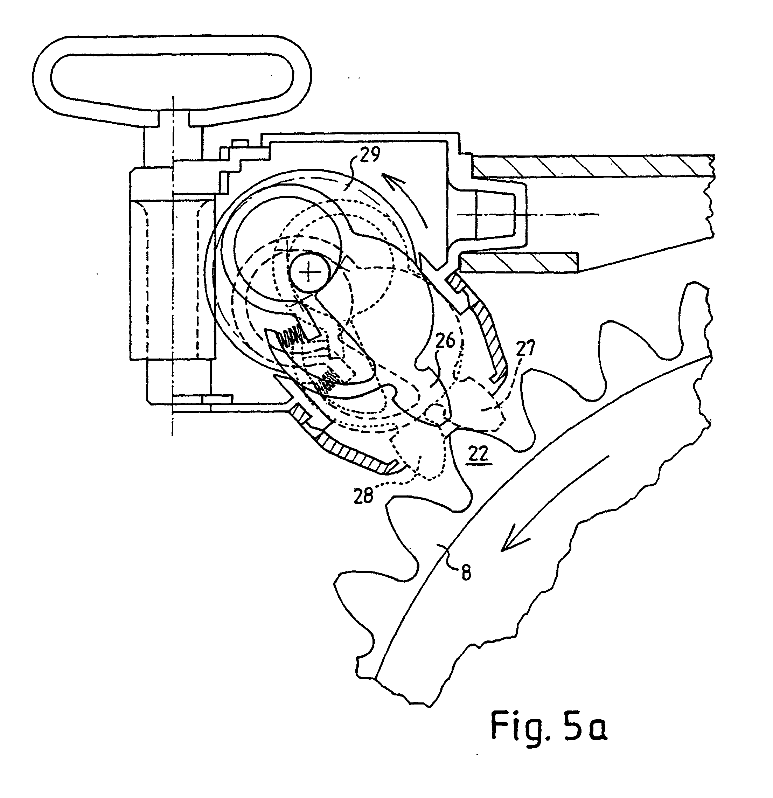

In Fig. 4 ist das Schneckenrad 17 mit den an beiden Stirnseiten exzentrisch um 180° versetzt angeordneten Antriebszapfen 15, 16 nochmals als Einzelheit dargestellt. Wie bereits oben erwähnt erfolgt die Lagerung des Schneckenrads 17 über Lagerachsen 18, die aus den exzentrisch angeordneten Antriebswellen 15, 16 herausragen.

Wie insbesondere aus Fig. 1 ersichtlich, sind die Spitzen 11, 12 der Antriebshebel 9, 10 nach unten abwärts gerichtet, so daß Flüssigkeit, die durch das Eintauchen des Zahnkranzes 8 in ein Flüssigkeitsbad zu den Hebelspitzen 11, 12 gefördert wird, wieder hinunter zum Zahnkranz 8 abläuft. Die einzigen Stellen, die also mit der Flüssigkeit des Bades in Verbindung kommen können, sind lediglich die zahnförmigen Spitzen 11, 12 der Antriebshebel 9, 10. Es besteht somit keine Gefahr, daß die restlichen Komponenten des Antriebs in irgendeiner Phase des Betriebs des Geräts mit der Badflüssigkeit in Berührung kommen. Eine Zerlegung des Geräts für Reinigungsarbeiten ist für den Laien in einfacher Weise möglich, da mit Abnahme des Deckels (nicht dargestellt) sich nur noch die Plattenstapel 1, 2 in der Wanne (nicht dargestellt) befinden.

Der dargestellte Antrieb für einen Plattenstapel 1, 2 läßt sich auch außerhalb eines erfindungsgemäßen Luftbefeuchtungsgeräts einsetzen. Das dargestellte System läßt sich ohne weiteres auf jeden rotierenden Gegenstand anwenden, bei dem ein erfindungsgemäßes Antriebssystem Vorteile bietet. Dies kann insbesondere bei chemischen Produktionsanlagen der Fall sein, wo ebenfalls häufig Walzen, Trommeln, Wärmetauscher oder dergleichen innerhalb eines Flüssigkeitsbades drehen müssen und somit sich ebenfalls das Problem stellt, daß der Antrieb nicht oder nur punkutell mit der gegebenenfalls aggressiven Flüssigkeit in Berührung kommen darf. Auch kann das erfindungsgemäße Antriebsprinzip vorteilhaft angewendet werden, wenn eine sehr große Übersetzung zwischen der Antriebswelle und dem rotierenden Gegenstand zu realisieren ist.

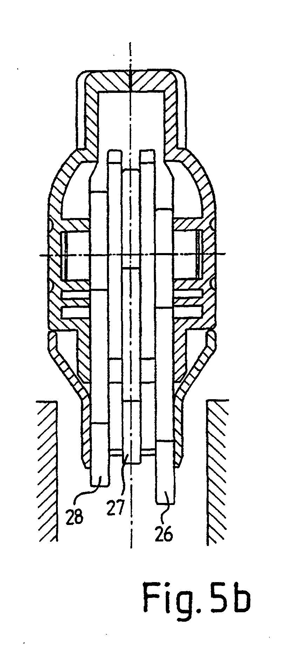

In Fig. 5a und b ist eine Variante eines erfindungsgemäßen Antriebs dargestellt, bei dem anstatt zweier Antriebshebel drei Antriebshebel 26, 27, 28 verwendet werden. In entsprechender Weise sind Antriebszapfen auf einem Schneckenrad 29 nicht um 180° sondern um 120° exzentrisch versetzt. Durch diese Ausführungsform, die in Fig. 5b zur Verdeutlichung der Lage der Antriebshebel 26, 27, 28 im Längsschnitt dargestellt ist, läßt sich ein noch verbesserter Gleichlauf erzielen.

### Bezugszeichenliste:

- 1: Plattenstapel
- 2: Plattenstapel
- 3: Antrieb
- 4: Achse
- 5: Achse
- 6: elastische Kuppel
- 7: Hebelgetriebe
- 8: Zahnkranz
- 9: Antriebszapfen
- 10: Antriebszapfen
- 11: zahnförmige Spitze
- 12: zahnförmige Spitze
- 13: Aufnahmeöffnung
- 14: Aufnahmeöffnung
- 15: Antriebszapfen
- 16: Antriebszapfen
- 17: Schneckenrad
- 18: Achse
- 19: Schnecke
- 20: Führungsfläche
- 21: Führungsfläche
- 22: Zahn
- 23: Rahmen
- 24: Zapfen
- 25: Druckfeder
- 26: Antriebshebel
- 27: Antriebshebel
- 28: Antriebshebel
- 29: Schneckenrad

## Patentansprüche

1. Antrieb für einen rotierenden Gegenstand, wie eine Walze, Welle, Platte oder dergleichen, insbesondere für einen Duftverdunster, einen Luftbefeuchter oder ein Luftreinigungsgerät oder dergleichen, mit wenigstens einem beweglich gelagerten Hebel, einem Zahnrad (8), das drehfest mit dem rotierenden Gegenstand verbunden ist sowie einem Hebelgetriebe (7) für eine in das Zahnrad eingreifende und mitnehmende Bewegung des Hebels zur Drehung des Zahnrades, wobei der Hebel zum Eingriff in die Zähne des Zahnrades eine Spitze (11, 12) aufweist, **dadurch gekennzeichnet, daß** die Spitze (11, 12) in Form eines Zahnes ausgebildet ist, wobei die Form diese Zahnes und die der Zähne des Zahnrades (8) sowie das Hebelgetriebe (7) derart aufeinander abgestimmt sind, daß die Zahnflanken des Zahnes (11, 12) am wenigstens einen Hebel (9, 10) und des jeweils im Eingriff stehenden Zahnes (22) am Zahnrad bei der mitnehmenden Bewegung aufeinander abwälzen.

2. Antrieb nach Anspruch 1, **dadurch gekennzeichnet, daß** die Form des Zahnes (11, 12) am wenigstens einen Hebel (9, 10) und die der Zähne (22) des Zahnrades (8) sowie das Hebelgetriebe (7) derart aufeinander abgestimmt sind, daß die Zahnflanken des Zahnes am wenigstens einen Hebel (9, 10) und des jeweils im Eingriff stehenden Zahnes (21) am Zahnrad (8) bei der mitnehmenden einen Berührungspunkt aufweisen, in dem die Zahnflanken im Wesentlichen ohne radiales Gleiten aufeinander abrollen.

3. Antrieb nach einem der Ansprüche 2 oder 3 , **dadurch gekennzeichnet, daß** die Form des Zahnes am Hebel (9, 10) und der Zähne (22) des Zahnrades (8) nach den Regeln der Verzahnung einer Evolventen-, Zykloiden-, Kreisbogen-, oder Triebstockverzahnung ausgebildet ist.

4. Antrieb nach einem der vorhergehenden Ansprüche, **dadurch gekennzeichnet, daß** das Hebelgetriebe (7) zur Schubführung des Hebels (9, 10) Führungsflächen (20, 21) beinhaltet.

5. Antrieb nach Anspruch 4, **dadurch gekennzeichnet, daß** das Hebelgetriebe (7) einen Exzenterantrieb für den Hebel (9, 10) umfaßt.

6. Antrieb nach Anspruch 5, **dadurch gekennzeichnet, daß** der Exzenterantrieb eine Antriebswelle (15, 16) für den Hebel aufweist, die exzentrisch auf einem Zahnrad angeordnet ist.

7. Antrieb nach Anspruch 6, **dadurch gekennzeichnet, daß** das Zahnrad ein Schneckenrad (17) ist, in das eine Schnecke (19) eingreift.

8. Antrieb nach Anspruch 7, **dadurch gekennzeichnet, daß** die Schnecke (19) über eine elastische Kupplung mit der Antriebswelle eines Motors verbunden ist.

9. Antrieb nach einem der vorhergehenden Ansprüche, **dadurch gekennzeichnet, daß** zwei, drei oder mehr Hebel (9, 10; 26, 27, 28) vorhanden sind.

10. Antrieb insbesondere nach einem der vorhergehenden Ansprüche 4 bis 9, **dadurch gekennzeichnet, daß** der Hebel (9, 10) zwischen den Führungsflächen (20, 21) des Hebelgetriebes (7) elastisch gelagert ist.

11. Antrieb nach Anspruch 10, **dadurch gekennzeichnet, daß** zur elastischen Lagerung des Hebels (9, 10) zwischen den Führungsflächen (20, 21) wenigstens ein Teil der von den Führungsflächen erfaßten Außenkontur des Hebels elastisch nachgiebig ist.

12. Antrieb nach Anspruch 11, **dadurch gekennzeichnet, daß** zur Realisierung einer elastisch nachgiebigen Außenkontur des Hebels (9, 10) die Außenkontur als schmaler Rahmen (23) ausgebildet ist.

13. Antrieb nach Anspruch 12, **dadurch gekennzeichnet, daß** der Rahmen (23) unterbrochen ist, wobei in die Unterbrechungsstelle ein Federelement (25) eingefügt ist.

14. Antrieb nach einem der Ansprüche 10 bis 13, **dadurch gekennzeichnet, daß** der Bereich der Außenkontur des Hebels, der sich zur Aufnahme der wesentlichen Kraft für eine Weiterbewegung des Zahnrads an einer Führungsfläche (20) abstützt, nicht elastisch nachgiebig ist.

## Claims

1. Drive mechanism for a rotating object, such as a roller, shaft, plate or the like, in particular for a fragrance vaporiser, an air humidifier or an air purifier or the like, with at least one movably mounted lever, a gear wheel (8) non-rotatably connected to the rotating object, and with a lever mechanism (7) for a movement of the lever engaging and entraining the gear wheel to rotate the gear wheel, the lever having a tip (11, 12) to engage in the teeth of the gear wheel, **characterised in that** the tip (11, 12) is designed in the form of a tooth, the form of this tooth and that of the teeth of the gear wheel (8) and the lever mechanism (7) being coordinated with each other in such a way that the tooth flanks of the tooth (11, 12) on the at least one lever (9, 10) and of the respectively engaged tooth (22) on the gear wheel roll off each other during the entraining movement.

2. Drive mechanism according to claim 1, **characterised in that** the form of the tooth (11, 12) on at least one lever (9, 10) and that of the teeth (22) of the gear wheel (8) and the lever mechanism (7) are coordinated with one another in such a way that the tooth flanks of the tooth on at least one lever (9, 10) and of the respectively engaged tooth (21) on the gear wheel (8) during the entraining movement have a contact point in which the tooth flanks roll off each other substantially without radial sliding.

3. Driving mechanism according to either of claims 2 or 3, **characterised in that** the form of the tooth on the lever (9, 10) and of the teeth (22) of the gear wheel (8) is designed according to the rules of toothing of an involute, cycloidal, round-flank, or pin wheel toothing.

4. Drive mechanism according to any one of the preceding claims, **characterised in that** the lever mechanism (7) contains guide faces (20, 21) for the thrust guidance of the lever (9, 10).

5. Drive mechanism according to claim 4, **characterised in that** the lever mechanism (7) comprises an eccentric drive for the lever (9, 10).

6. Drive mechanism according to claim 5, **characterised in that** the eccentric drive comprises a drive shaft (15, 16) for the lever, which drive shaft (15, 16) is arranged eccentrically on a gear wheel.

7. Drive mechanism according to claim 6, **characterised in that** the gear wheel is a worm wheel (17), in which a worm (19) engages.

8. Drive mechanism according to claim 7, **characterised in that** the worm (19) is connected to the drive shaft of a motor via a resilient coupling.

9. Drive mechanism according to any one of the preceding claims, **characterised in that** there are two, three or more levers (9, 10; 26, 27, 28).

10. Drive mechanism, in particular according to any one of the preceding claims 4 to 9, **characterised in that** the lever (9, 10) is resiliently mounted between the guide faces (20, 21) of the lever mechanism (7).

11. Drive mechanism according to claim 10, **characterised in that** at least part of the external contour of the lever grasped by the guide faces is resiliently flexible for resilient mounting of the lever (9, 10) between the guide faces (20, 21).

12. Drive mechanism according to claim 11, **characterised in that** the external contour is designed as a narrow frame (23) to produce a resiliently flexible external contour of the lever (9, 10).

13. Drive mechanism according to claim 12, **characterised in that** the frame (23) is interrupted, a spring element (25) being inserted into the interruption point.

14. Drive mechanism according to any one of claims 10 to 13, **characterised in that** the region of the external contour of the lever which is supported on a guide face (20) to receive the substantial force for further movement of the gear wheel, is not resiliently flexible.

## Revendications

1. Entraînement pour un objet rotatif, comme un cylindre, un arbre, un disque ou analogue, en particulier pour un évaporateur de parfum, un humidificateur d'air ou un appareil de purification d'air ou analogue, comportant au moins un levier monté de façon mobile, une roue dentée (8), qui est reliée de façon solidaire en rotation avec l'objet rotatif, ainsi qu'une transmission de levier (7) pour un mouvement d'entraînement, et engageant la roue dentée, du levier pour la rotation de la roue dentée, le levier présentant, pour l'engagement dans les dents de la roue dentée, une pointe (11, 12),
**caractérisé en ce que** la pointe (11, 12) est réalisée sous forme d'une dent, la forme de cette dent et celle des dents de la roue dentée (8), ainsi que la transmission de levier (7), étant accordées les unes aux autres de sorte que les flancs de la dent (11, 12) sur au moins un levier (9, 10) et de la dent (22), qui est à chaque fois en engagement, sur la roue dentée, lors du mouvement d'entraînement, roulent l'un sur l'autre.

2. Entraînement selon la revendication 1,
**caractérisé en ce que** la forme de la dent (11, 12) sur au moins un levier (9, 10) et celle des dents (22) de la roue dentée (8), ainsi que la transmission de levier (7), sont accordées les unes aux autres de sorte que les flancs de la dent sur au moins un levier (9, 10) et de la dent (22), qui est à chaque fois en engagement, sur la roue dentée (8) présentent, lors du mouvement d'entraînement, un point de contact, où les flancs des dents roulent l'un sur l'autre généralement sans glissement radial.

3. Entraînement selon une des revendications 2 ou 3,
**caractérisé en ce que** la forme de la dent sur le levier (9, 10) et des dents (22) de la roue dentée (8) est réalisée selon les règles de la denture d'une denture à développante, à cycloïde, à arc de cercle, ou à fuseaux.

4. Entraînement selon une des revendications précédentes,
**caractérisé en ce que** la transmission de levier (7) pour le guidage de poussée du levier (9, 10) présente des surfaces de guidage (20, 21).

5. Entraînement selon la revendication 4,
**caractérisé en ce que** la transmission de levier (7) comporte un entraînement à excentrique pour le levier (9, 10).

6. Entraînement selon la revendication 5,
**caractérisé en ce que** l'entraînement à excentrique présente un arbre d'entraînement (15, 16) pour le levier qui est agencé de façon excentrique sur une roue dentée.

7. Entraînement selon la revendication 6,
**caractérisé en ce que** la roue dentée est une roue à denture hélicoïdale (17) dans laquelle s'engage une vis sans fin (19).

8. Entraînement selon la revendication 7,
**caractérisé en ce que** la vis sans fin (19) est reliée à l'arbre d'entraînement d'un moteur par l'intermédiaire d'un accouplement élastique.

9. Entraînement selon une des revendications précédentes,
**caractérisé en ce que** deux, trois ou plus leviers (9, 10 ; 26, 27, 28) sont prévus.

10. Entraînement en particulier selon une des revendications précédentes 4 à 9,
**caractérisé en ce que** le levier (9, 10) est monté de façon élastique entre les surfaces de guidage (20, 21) de la transmission de levier (7).

11. Entraînement selon la revendication 10,
**caractérisé en ce que**, pour le montage élastique du levier (9, 10), entre les surfaces de guidage (20, 21), au moins une partie du contour externe, saisi par les surfaces de guidage, du levier est élastiquement souple.

12. Entraînement selon la revendication 11,
**caractérisé en ce que**, pour réaliser un contour externe élastiquement souple du levier (9, 10), le contour externe est réalisé comme cadre étroit (23).

13. Entraînement selon la revendication 12,
**caractérisé en ce que** le cadre (23) est interrompu, un élément élastique (25) étant introduit dans l'endroit d'interruption.

14. Entraînement selon une des revendications 10 à 13,
**caractérisé en ce que** la zone du contour externe du levier, qui s'appuie contre une surface de guidage (20) pour recevoir la force principale pour un mouvement ultérieur de la roue dentée, n'est pas élastiquement souple.
